(19) Europäisches Patentamt
European Patent Office
Office européen des brevets

(11) Veröffentlichungsnummer: **0 058 246**
**B1**

(12) **EUROPÄISCHE PATENTSCHRIFT**

(45) Veröffentlichungstag der Patentschrift: **02.03.88**

(51) Int. Cl.⁴: **C 07 D 307/20,**
C 07 D 493/04, C 07 C 31/26

(21) Anmeldenummer: **81109423.4**

(22) Anmeldetag: **30.10.81**

# (54) Verfahren zur Herstellung von Polyolgemischen und neue Polyolgemische.

(30) Priorität: **05.11.80 DE 3041626**

(43) Veröffentlichungstag der Anmeldung:
**25.08.82 Patentblatt 82/34**

(45) Bekanntmachung des Hinweises auf die Patenterteilung:
**02.03.88 Patentblatt 88/09**

(84) Benannte Vertragsstaaten:
**BE CH DE FR GB IT LI NL SE**

(56) Entgegenhaltungen:
**US-A-3 454 603**

(73) Patentinhaber: **CPC INTERNATIONAL INC.**
**International Plaza P.O. Box 8000**
**Englewood Cliffs New Jersey 07632 (US)**

(72) Erfinder: **Feldmann, John, Dr. Dipl.-Chem.**
**Grenzstrasse 151**
**D-4150 Krefeld (DE)**
Erfinder: **Koebernik, Hubert, Dr. Dipl.-Chem.**
**Hohenzollernstrasse 76**
**D-4150 Krefeld (DE)**
Erfinder: **Woelk, Hans-Ulrich, Dr. Dipl.-Chem.**
**Friedrich Kirstenstrasse 3**
**D-2000 Hamburg 65 (DE)**

(74) Vertreter: **Deufel, Paul, Dr. et al**
**Patentanwälte Müller-Boré, Deufel, Schön, Hertel, Lewald, Otto Isartorplatz 6 Postfach 26 02 47**
**D-8000 München 26 (DE)**

**EP 0 058 246 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren der eingangs bezeichneten Art und danach erhältliche neue Polyolgemische.

$C_4$—$C_6$-Zuckeralkohole, insbesondere Hexite, Anhydrozuckeralkohole der Formel I und Gemische dieser Polyole sind seit langem bekannte Produkte, die u.a. als Ausgangsmaterialien oder Zwischenprodukte für chemische Synthesen, insbesondere von chiralen Feinchemikalien, Emulgatoren auf Esterbasis, Polyerster- und Alkydharzen, sowie insbesondere von Polyurethanen verwendet werden. Für den Einsatz als Polyolkomponente bei der Herstellung synthetischer Harze ist außer der Art der Polyole bzw. Polyolgemische vor allem deren Hydroxylzahl von wesentlicher Bedeutung, die theoretisch zwischen 1848 für reine Hexite und 768 für reine Dianhydrohexite variiert werden kann.

Während praktisch reine $C_4$—$C_6$-Zuckeralkohole, insbesonders Hexite leicht und in nahezu quantitativer Ausbeute durch katalytische Hydrierung der entsprechenden Zuckerarten, insbesondere Hexosen, zugänglich sind, erhält man bei den bekannten Verfahren zur Herstellung von Verbindungen der Formel I bzw. diese enthaltenden Polyolgemischen durch Wasserabspaltung aus Zuckeralkoholen bei erhöhten Temperaturen in Gegenwart saurer Katalysatoren nur Gemische mit recht begrenztem Gehalt an Polyolen der Formel I, z.B., ausgehend von Hexiten, höchstens etwa 66% Di- und insbesondere höchstens etwa 40% Monoanhydrohexit, wobei zudem das Mengenverhältnis, in dem die verschiedenen Polyole im Reaktionsgemisch vorliegen, für eine bestimmte Hydroxylzahl vor allem im Bereich mittlerer und niedriger Hydroxylzahlen durch die Wahl der Verfahrensparameter, insbesondere Katalysator, Temperatur, Reaktionsmedium und/oder -zeit allenfalls geringfügig verändert werden kann.

Hinzu kommt, daß vergleichsweise hohe Di- und insbesondere Monoanhydrohexitausbeuten nach dem Stand der Technik nur bei der Verwendung sogenannter homogener, d. h. im Reaktionssystem molekulardispers verteilter Katalysatoren, wie Salz- oder Schwefelsäure, erzielt werden, die recht korrosiv sind und zur Bildung von Nebenprodukten, insbesondere Estern der Katalysatorsäure, z. B. Halogenierungsprodukten führen, sowie nach der Umsetzung eine Neutralisation mit nachfolgender Entsalzung des Reaktionsgemischs erfordern (Carbohydrate Chemistry, Vol. II, 191—198 (1963)). Ein Verfahren dieser Gattung wird auch in der US—PS 3 454 603 beschrieben. Die gleichfalls in diesem Zusammenhang bekannte Mitverwendung von organischen Lösungsmitteln zur Entfernung des Reaktionswassers durch azeotrope Destillation ergibt nur eine geringe Verbesserung der Anhydrohexitausbeuten.

Einige der Nachteile der Verfahren mit im Reaktionsgemisch gelösten Säurekatalysatoren, wie die Korrosionsprobleme und die Bildung excessiver Mengen nur schwer abtrennbarer veresterter Nebenprodukte, lassen sich zwar in bekannter Weise durch die Verwendung heterogener Katalysatoren, nämlich (bestimmter) stark saurer Kationenaustauscherharze vermeiden, jedoch muß dies durch einige andere Nachteile, insbesondere noch schlechtere Ausbeuten an Anhydrohexiten von nur bis zu 35% und die Notwendigkeit, organische Lösungsmittel als Reaktionsmedium und Schleppmittel für die Entfernung von Wasser durch Azeotropdestillation zu verwenden, erkauft werden (vgl. Ropuszynski und Mit. in Przem. Chem. 1969, 48 (11), 665—8 und Weisleder und Mit. in Carbohydr. Res. *79*, 133—141 (1980)). Was die Notwendigkeit der Verwendung sorgfältig ausgewählter organischer Lösungsmittel(gemische) betrifft, wird in der letzgenannten Arbeit darauf hingewiesen, daß bei dem — zum Vergleich durchgeführten — Versuch 1,4;3,6-Dianhydro-D-sorbit aus Sorbit durch "trockene Destillation mit dem sauren Harz" herzustellen eine noch mehrfach geringere Ausbeute von nur 5% erzielt wurde.

Nach dem Stand der Technik kann somit nur ein verhältnismäßig kleiner Ausschnitt aus dem breiten Spektrum der fraglichen Polyolgemische als unmittelbares Reaktionsprodukt hergestellt werden, während man den größten Teil dieser Gemische, und zwar insbesondere diejenigen mit Hydroxylzahlen im mittleren und unteren Teil des theoretischen Bereiches nur dadurch erhalten könnte, daß man aus den Reaktionsproduktgemischen durch aufwendige Trennverfahren, wie fraktionierende Kristallisation oder Destillation und insbesondere Chromatographie, mit Mono- und/oder Dianhydrohexit(en) angereicherte Fraktionen abtrennt und diese gegebenenfalls zu Produkten der jeweils gewünschten Hydroxylzahl und Polyolzusammensetzung verschneidet. Dies wäre angesichts der bestimmungsgemäßen Verwendung zur Herstellung von Kunstharzen zu aufwendig und kostspielig.

Der Erfindung lag daher die Aufgabe zugrunde, ein Verfahren zu schaffen, das die vorstehenden Nachteile des Standes der Technik überwindet und es insbesondere ermöglicht, ein breites Spektrum von — teilweise neuen — Polyolgemischen der fraglichen Art, das bezüglich Hydroxylzahl und Mengenverhältnis von Zuckeralkohol(en) zu Monoanhydrozuckeralkohol(en) zu Dianhydrohexit(en) die theoretischen Bereiche nahezu vollständig abdeckt, unmittelbar durch Wasserabspaltung in Gegenwart saurer Katalysatoren herzustellen.

Diese Aufgabe wird erfindungsgemäß in der im Hauptanspruch gekennzeichneten Weise ausgehend von der Erkenntnis gelöst, daß Wasser, das bei den bekannten Verfahren ausnahmslos laufend möglichst vollständig entfernt werden muß, überraschenderweise bei Umsetzungen der fraglichen Art nicht nur nicht stört, sondern es sogar ermöglicht, Reaktionsproduktgemische mit höheren Gehalten an Monoanhydrozuckeralkohol(en) und/oder Dianhydrohexit(en) zu erhalten, als dies nach dem Stand der Technik in weitaus wasserärmeren Reaktionssystemen möglich war, wenn man heterogene Katalysatoren verwendet und die Wasserkonzentration in dem beanspruchten Bereich hält.

Die Erfindung betrifft demgemäß ein Verfahren zur Herstellung von mindestens zwei Polyole verschiedenen Molekulargewichts aus den Gruppen der $C_4$—$C_6$-Zuckeralkohole und $C_4$—$C_6$-Anhydrozuckeralkohole der Formel

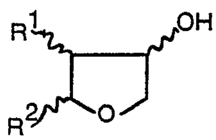

(I)

in der $R^1$ eine Hydroxylgruppe oder das Äthersauerstoffatom eines zweiten Oxacyclopentanrings und $R^2$ ein Wasserstoffatom oder ein Hydroxymethyl- oder 1,2-Dihydroxyäthylrest ist oder zusammen mit $R^1$ und den Kohlenstoffatomen, an die $R^1$ und $R^2$ gebunden sind, einen zweiten Oxacyclopentanring bildet, enthaltenden Polyolgemisches, mit der Maßgabe, daß mindestens ein Polyol der Formel I vorhanden ist, durch Wasserabspaltung aus $C_4$—$C_6$-Zuckeralkohol(en) bei erhöhter Temperatur in flüssiger Phase und in Gegenwart eines stark-sauren heterogenen Katalysators. Dieses Verfahren besteht darin, daß man die Umsetzung

a) in Gegenwart von Wasser in einer Menge, die mindestens dem bei der Umsetzung frei werdenden Reaktionswasser und höchstens 120 Gew.-%, bezogen auf das Gewicht des eingesetzten Zuckeralkohol(gemisch)s, zugesetztem Wasser (Fremdwasser) entspricht,

b) bei einer Temperatur zwischen 60 und 200°C und

c) mit einer (durchschnittlichen) Reaktionszeit, die so gewählt ist, daß die Hydroxylzahl des erhaltenen Polyolgemisches in einem Bereich von 810 bis 1800 liegt und das Molverhältnis von Zuckeralkohol(en) zu Monoanhydrozuckeralkohol(en) und/oder Dianhydrohexit(en) mindestens 1:9 beträgt, durchführt.

Vorzugsweise wird die Umsetzung solange fortgeführt, bis die Hydroxylzahl des erhaltenen Polyolgemisches in einem Bereich von 900 bis 1600, vorzugsweise 1100 bis 1500, und insbesondere 1150 bis 1400 liegt.

Es ist ferner vorzuziehen, die Umsetzung in Gegenwart von Wasser in einer Menge durchzuführen, die, dem Reaktionswasser zuzüglich 0,1 bis 100, vorzugsweise 1 bis 70 und insbesondere 3 bis 50 Gew.-% Fremdwasser, bezogen auf eingesetzte(n) Zuckeralkohol(e), entspricht.

Vorzugsweise wird erfindungsgemäß ein hexithaltiges Ausgangsmaterial verwendet, wobei man das zumindest in einer Phase, insbesondere der Anfangsphase der Umsetzung, auf einen einer Fremdwasserkonzentration von 1 bis 100, vorzugsweise 3 bis 70 und insbesondere 8 bis 50 Gew.-%, bezogen auf eingesetzte(n) Zuckeralkohol(e), entsprechenden Wassergehalt eingestellte Reaktionsgemisch so lange reagieren läßt, bis dessen Monoanhydrozuckeralkoholgehalt, bezogen auf Polyoltrockensubstanz, 35 bis 90, vorzugsweise 40 bis 85 und insbesondere 50 bis 80 Gew.-% beträgt und das Gewichtsverhältnis von Monoanhydro- zu Dianhydrohexit(en) in einem Bereich von 9:1 bis 1:2,8, vorzugsweise 4:1 bis 1:2 und insbesondere 2:1 bis 1:1 liegt.

Das Verfahren der Erfindung ermöglicht es, durch entsprechende Wahl der Reaktionsbedingungen, insbesondere der Wasserkonzentration und der Reaktionszeit Polyolgemische mit Monoanhydrozuckeralkoholgehalten, insbesondere Monoanhydrohexitgehalten von etwa 5 bis 80 Gew.-% und Dianhydrohexitgehalten von etwa 90 bis 5 Gew.-%, jeweils bezogen auf Polyoltrockensubstanzgewicht, herzustellen.

Da der Verlauf der Umsetzung und die Zusammensetzung des Reaktionsgemisches von einer Reihe von Faktoren, wie der Zusammensetzung des Ausgangsmaterials (außer reinen Zuckeralkoholen können erfindungsgemäß auch Zuckeralkoholgemische verwendet werden), dem Katalysator und der Reaktionstemperatur sowie insbesondere dem Wassergehalt des Reaktionsgemisches und der Reaktionsdauer abhängen, läßt sich natürlich keine Auswahlregel geben, aufgrund welcher die für jeden Einzelfall optimalen Bedingungen zahlenmäßig exakt vorausberechnet werden können, jedoch ist es dem Fachmann ohne weiteres gegeben, im Rahmen der Lehre des Hauptanspruchs für jedes gewünschte Polyolgemisch zweckmäßige Verfahrensbedingungen durch Variation des Wassergehalts und der Reaktionszeit anhand einiger orientierender Versuche unter Beachtung folgender Grundsätze zur ermitteln:

Die Reaktionsgeschwindigkeiten nehmen — wie üblich — mit steigender Temperatur rasch zu, wobei jedoch einer Reaktionsbeschleunigung bzw. Reaktionszeitverkürzung durch Reaktionstemperaturerhöhung durch die Temperaturbeständigkeit der Ausgangsmaterialien, Reaktionsprodukte und/oder Katalysatoren Grenzen gesetzt sind. Die Reaktionstemperatur wird daher in der Regel in einem Bereich von 80 bis 170, vorzugsweise 95 bis 160 und insbesondere 105 bis 145°C gehalten. Wie aus den Beispielen ersichtlich, haben sich bei der Verwendung von mit Divinylbenzol vernetzten Polystyrolsulfonsäure-Kationenaustauscherharzen in der $H^+$-Form als Katalysator Temperaturen von 120 bis 145°C besonders bewährt.

Wasser hemmt in steigender Konzentration die Reaktionsgeschwindigkeit, und zwar bei der Verwendung von $C_6$-Zuckeralkoholen, sowohl die der Umsetzung von Hexiten in Monoanhydrohexite als auch die der Bildung von Dianhydrohexiten aus Monoanhydrohexiten, wobei überraschenderweise einerseits die letztgenannte Reaktion offensichtlich wesentlich stärker verzögert wird als die erstgenannte und andererseits entgegen dem im Stand der Technik, der durchwegs eine möglichst weitgehende Entfernung selbst des Reaktionswassers als unabdingbare Voraussetzung für die Erzielung auch nur einigermäßen akzeptabler Anhydrozuckeralkoholausbeuten

lehrt, vertretenen Vorurteil weder bezüglich der Umsetzung ovn Hexiten zu Monoanhydrohexiten noch sogar hinsichtlich der Bildung von Di- durch Wasserabspaltung aus Monoanhydrohexiten das Reaktionsgleichgewicht so wesentlich zu Ungunsten der wasserärmeren Verbindung verschoben wird, daß keine akzeptablen Anhydrohexitausbeuten mehr zu erhalten wären; die nach dem Verfahren der Erfindung in Gegenwart relativ hoher Wasserkonzentration erreichbaren Anhydrohexitausbeuten liegen im Gegenteil sowohl bei Mono- (bis zu etwa 80 Gew.-% und mehr) als insbesondere auch bei Dianhydrohexiten (bis zu etwa 90 Gew.-% und mehr) deutlich über den in der Literatur für praktische wasserfreie Systeme genannten Höchstwerten von etwa 40 bzw. 66 Gew.-%. Dies wirkt sich beim Verfahren der Erfindung so aus, daß in Reaktionsgemischen ohne oder mit relativ geringem Fremdwasserzusatz von einigen wenigen Prozenten zunächst der Mono- und kurz darauf auch der Dianhydrohexitgehalt rasch anzusteigen beginnt, wobei mit zunehmender Reaktionsdauer der Dianhydrohexitgehalt laufend und verhältnismäßig rasch weiter bis auf etwa 90 Gew.-% steigt und bereits vor dem weitgehenden Verbrauch des eingesetzten Hexits beachtliche Werte annimmt, der Monoanhydrohexitgehalt degenen schon bald einen Höchstwert von etwa 40 Gew.-% erreicht und im weiteren Verlauf der Umsetzung ziemlich rasch wieder auf Werte im Bereich von nur 5 bis 10 Gew.-% absinkt (vgl. Fig. I), während sich in Reaktionsgemischen mit höheren Fremdwasserzusätzen die Anhydrohexitbildung einerseits insgesamt verlangsamt und andererseits so verändert, daß ab einem Fremdwassergehalt von etwa 8 bis 10 Gew.-% der Monoanhydrohexitgehalt bis zum weitgehenden Verbrauch des eingesetzten Hexits laufend weiter steigt, wobei Werte von bis zu 80 Gew.-% und fallweise sogar darüber erreicht werden können, und die Bildung beachtlicher Dianhydrohexitmengen erst relativ spät einsetzt (vgl. z. B. Fig. II). Für das Verfahren der Erfindung folgt aus dem vorstehenden Reaktionsverlauf, daß zur Herstellung von Polyolgemischen mit relativ geringem Monoanhydrohexitgehalt (bei gleicher Hydroxylzahl) die Umsetzung zweckmäßig in Reaktionssystemen ohne oder mit nur geringem Fremdwassergehalt von bis zu etwa 10 Gew.-% durchgeführt werden soll, während sich zur Herstellung von Polyolgemischen mit höhrerem Monoanhydrohexitgehalt die Anwendung höherer Fremdwasserzusätze von mehr als etwa 5 und insbesondere mehr als etwa 10 Gew.-% empfiehlt, und zwar insbesondere dann, wenn die gewünschte Hydroxylzahl im mittleren oder unteren Teil des beanspruchten Bereichs liegt und/oder der Monoanhydrohexitgehalt des Polyolgemischs über etwa 40 und insbesondere über 50 Gew.-% betragen soll.

In der Regel sind Fremdwasserzusätze von etwa 0,1 bis 100, vorzugsweise 1 bis 70 und insbesondere 3 bis 50 Gew.-%, bezogen auf das Gewicht des Hexiteinsatzes, zweckmäßig.

Der Fremdwassergehalt des Reaktionsgemisches läßt sich beim Verfahren der Erfindung zweckmäßigerweise dadurch konstant halten, daß man unter Rückflußbedingungen arbeitet.

Fallweise, und zwar insbesondere dann, wenn Polyolgemische mit extrem niedrigem Zuckeralkoholgehalt und Hydroxylzahlen im mittleren bis unteren Teil des beanspruchten Bereichs, also hohen Gehalten an Monoanhydrozuckeralkohol(en) und/oder Dianhydrohexit(en) hergestellt werden sollen, ist es zweckmäßig, den Wassergehalt des Reaktionsgemischs im Laufe der Umsetzung kontinuierlich oder schrittweise zu verändern, wobei es sich in der Regel empfiehlt, die Reaktion mit einem relativ hohen Wassergehalt zu beginnen, die Wasserkonzentration zu verringern, wenn der Zukkeralkoholgehalt des Reaktionsgemischs bis in die Nähe des gewünschten Endwerts gefallen ist, und dann bei dem verringerten Wassergehalt bis zum Erreichen des gewünschten Verhältnises von Monoanhydrozuckeralkohol(en) zu Dianhydrohexit(en) weiterlaufen zu lassen. Diese Ausführungsform der Erfindung ist insbesondere deswegen vorteilhaft, weil sie nicht nur Polyolgemische mit bislang nicht erreichbaren Monoanhydrohexitgehalten im Bereich von 35 bis 90, vorzugsweise 40 bis 85 und insbesondere 50 bis 80 Gew.-% zugänglich macht, zu deren Erhalt, insbeonsdere dann, wenn zugleich ein hohes Verhältnis von Mono- zu Dianhydrohexit(en) angestrebt wird, in der Regel Fremdwasserkonzentrationen in einem Bereich von 1 bis 100, vorzugsweise 3 bis 70 und insbesondere 8 bis 50 Gew.-%, bezogen auf eigensetzte(n) Hexit(e), erforderlich sind, sondern gegenüber einer Arbeitsweise mit gleichbleibend niederer oder auch hoher Wasserkonzentration auch zu einer Verringerung der thermischen Belastung der temperaturempfindlichen Reaktionsgemischkomponenten und/oder einer Verkürzung der Gesamtreaktionszeit führt. Hierbei sei auch darauf hingewiesen, daß die erfindungsgemäß zu verwendenden Katalyusatoren teilweise eine doch recht begrenzte Temperaturbeständigkeit besitzen und es daher meist unzweckmäßig ist, sie vor dem Einsatz vollständig zu trocknen. Das durch feuchte Katalysatoren in das Reaktionssystem eingeschleppte Fremdwasser stört erfindungsgemäß aber zumindest bei der vorstehenden Ausführungsform selbst dann nicht, wenn Polyolgemische mit extrem niedrigen Zuckeralkohol- und/oder extrem hohen Dianhydrohexitgehalten angestrebt werden, die bei höheren Fremdwassergehalten nicht zu erreichen sind, da es problemlos im Verlauf der Umsetzung bis zum gewünschten Grad abgetrennt werden kann. Das gleiche gilt sinngemäß für etwaiges mit dem Ausgangsmaterial eingeschlepptes (überschüssiges) Fremdwasser.

Wie bereits erwähnt, kommen die Vorteile des Verfahrens der Erfindung vor allem bei der Herstellung von Polyolgemischen mit Hydroxylzahlen im unteren und insbesondere im mitteleren Teil des beanspruchten Bereiches und gegenüber dem Ausgangsmaterial stark verringertem Zuckeralkoholgehalt zum Tragen, weshalb man

erfindungsgemäß die Reaktion vorzugsweise so lange laufen läßt, bis die Hydroxylzahl des erhaltenen Polyolgemisches in einem Bereich von 900 bis 1600, vorzugsweise 1100 bis 1500 und insbesondere 1150 bis 1400 liegt und/oder sein (Rest-)Zuckeralkoholgehalt nur noch höchstens 40, vorzugsweise weniger als 20 und insbesondere nicht mehr als 10 Gew.-% beträgt.

In der Regel liegen die Reaktionszeiten beim Verfahren der Erfindung in einem Bereich von 0,1 bis 50 Stunden. Die im Einzelfall optimale Reaktionszeit wird zweckmäßigerweise durch Vorversuche ermittelt, wobei eine Vorauswahl des in Betracht kommenden engeren Bereiches meist anhand der beiliegenden Figuren I oder II erfolgen kann, in denen jeweils der Verlauf der Reaktionsgemischzusammensetzung bei für das Verfahren der Erfindung typischen Bedingungen (Verwendung von Sorbit als Ausgangsmaterial und eines startk-sauren makroporösen, mit 14% Divinylbenzol vernetzten Polystyrolsulfonsäure-Kationenaustauscherharzes in der $H^+$-Form als Katalysator und Anwendung einer Reaktionstemperatur von 125°C) bei einer Fremdwasserkonzentration von 1 Gew.-% (Fig. I) bzw. 70 Gew.-% (Fig. II) graphisch dargestellt ist.

Als heterogene Katalysatoren kommen für das Verfahren der Erfindung außer stark sauren Kationenaustauscherharzen im Prinzip alle an sich bekannten stark sauren festen Katalysatoren mit ausreichender Temperaturbeständigkeit und insbesondere "saure" Molekularsiebe, wie Zeolithe, und die vor allem in der Mineralölindustrie gebräuchlichen Crack- und/oder Hydrocrackkatalysatoren in Betracht, die für die Zwecke der Erfindung vor allem deswegen von Interesse sind, weil sie auch für die Herstellung von Hexiten durch katalytische Hydrierung der entsprechenden Zucker verbreitete Anwendung finden und somit den Weg für integrierte, ein- oder wenigstufige Verfahren zur Herstellung der fraglichen Polyolgemische aus Zuckern eröffnen.

Am besten haben sich bislang jedoch starksaure synthetische Kationenaustauscherharze und davon wiederum insbesondere mit Divinylbenzol vernetzte Polystyrolsulfonsäure-Kationenaustauscherharze bewährt. Dabei kommen sowohl sog. Gelharze, die nur schwach, also mit höchstens 4% Divinylbenzol vernetzt sein sollten, als auch makroporöse Harze in Betracht, die zweckmäßigerweise stark, d. h. mit mindestens 10% Divinylbenzol vernetzt sein sollten.

Der Katalysator wird beim Verfahren der Erfindung durch einfache Fest/Flüssig-Trennmethoden, z. B. Filtration, vom Reaktionsproduktgemisch getrennt und kann erfahrensgemäß ohne Aufbereitung mehrfach wiederverwendet werden.

Beim Verfahren der Erfindung empfiehlt es sich fallweise, nämlich insbesondere dann, wenn mit relativ hohen Reaktionstemperaturen und langen Reaktionszeiten gearbeitet wird, die Umsetzung in einer Inertgasatmosphäre durchzuführen (um Oxidationsreaktionen zu vermeiden, wobei man zweckmäßigerweise Inertgas durch das Reaktionsgemisch perlen läßt.

Weiter kann man beim Verfahren der Erfindung gewünschtenfalls organische, mit Wasser zumindest teilweise misch- und/oder azeotrop destillierbare (Hilfs-)Lösungsmittel mitverwenden, was insbesondere dann häufig zweckmäßig ist, wenn mit relativ niedrigen Wassergehalten unter Rückfluß gearbeitet wird.

Es sei ferner darauff hingewiesen, daß das Verfahren der Erfindung zwar häufig absatzweise durchgeführt wird, zur Herstellung größerer Mengen von Polyolgemischen bestimmter Zusammensetzung aber ohne weiteres auch nach an sich bekannten kontinuierlichen Methoden durchgeführt werden kann, wobei sich dann in der Regel eine zwei- oder mehrstufige Arbeitsweise empfiehlt.

Gegenstand der Erfindung sind such die mach dem erfindungsgemäßen Verfharen erhaltlichen neuen Polyolgemische, die sich durch ungewöhnlich hohe Mononahydrohexitgehalte von mindestens 43, vorzugsweise mindestens 50 und insbesondere mindestens 55 Gew.-% bei Hydroxylzahlen im Bereich von 1030 bis 1600 auszeichnen und nach bekannten Verfahren nicht als unmittelbare Reaktionsprodukte hätten hergestellt werden können.

Die Beispiele erläutern die Erfindung und deren Vorteile am Beispiel des Sorbits, der erfindungsgemäß als Ausgangsmaterial bevorzugt wird, weil er derzeit als einziger Hexit zu vertretbaren Preisen als technisches Produkte in praktisch unbegrenzten Mengen erhältich ist, sowie von durch katalytische Hydrierung von Hemicellulosehydrolysaten hergestellten $C_4$—$C_6$-Zuckeralkoholgemischen, die ein weiteres preiswertes und zu Polyolgemischen mit wertvollen Eigenschaften führendes Ausgangsmaterial zur Durchführung des erfindungsgemäßen Verfahrens darstellen.

Die erfindungsgemäß aus hydrierten Hemicellulosehydrolysaten erhältlichen Anhydrozuckeralkoholgemische mit einer Hydroxylzahl von 900 bis 1600 stellen neue Produkte dar.

Angaben in Prozent beziehen sich, soweit nicht ausdrücklich etwas anderes angegeben ist, jeweils auf das Gewicht.

Beispiel 1

In einem 1 Ltr. Dreihalskolben wurden 250 g Sorbit und 20 g eines stark sauren Ionenaustauschers in der $H^+$-Form (makroporöses Polystyrolsulfonsäureharz, mit 14% DVB-vernetzt) eine Stunde unter Rückflußbedingungen (125°C) gerührt. Der durch die benutzten Reagenzien eingeschleppte Fremdwassergehalt betrug etwa 2 g, entsprechend 0,8%, bezogen auf eingesetzten Sorbit. Die Reaktionslösung wurde vom Katalysator abfiltriert, man erhielt nach Abdestillieren des Reaktionswassers 233 g eines Polyolgemisches, das 79% Sorbit, 15% Monoanhydrosorbit und 6% Dianhydrosorbit enthielt. Die Hydroxylzahl des erhaltenen Gemisches betrug 1711.

Beispiel 2

Beispiel 1 wurde wiederholt, wobei jedoch abweichend davon erst nach einer Reaktionszeit von 3 Std. filtriert und eingeengt wurde. Man erhielt 214 g eines Polyolgemisches folgender Zusammensetzung: 31% Sorbit, 36% Monoanhydrosorbit, 33% Dianhydrosorbit. Die Hydroxylzahl wurde zu 1319 bestimmt.

Beispiel 3

Beispiel 2 wurde mit einer auf 4 Stunden verlängerten Reaktionszeit wiederholt. Es wurden 206 g eines Polyolgemischs aus 10% Sorbit, 40% Monoanhydrosorbit und 50% Dianhydrosorbit erhalten. Die Hydroxylzahl der Mischung betrug 1116.

Beispiel 4

Beispiel 3 wurde mit einer auf 5 Stunden verlängerten Reaktionszeit wiederholt. Nach Filtration und Destillation wurden 197 g eines Gemisches aus 1% Sorbit, 28% Monoanhydrosorbit und 71% Dianhydrosorbit mit der Hydroxylzahl 946 erhalten.

Beispiel 5

Beispiel 4 wurde mit einer auf 7 Stunden verlängerten Reaktionszeit wiederholt. Dabei wurde ein Polyolgemisch (192 g) aus 9% Monoanhydrosorbit und 91% Dianhydrosorbit mit einer Hydroxylzahl von 822 erhalten.

Beispiel 6

In einem 1 Ltr. Dreihalskolben wurden 250 g Sorbit mit 28 g Wasser und 20 g des unter Beispiel 1 beschriebenen Ionenaustauschers versetzt und 1 Stunde bei 125°C unter Rückflußbedingungen gerührt (Fremdwassergehalt 12%, bezogen auf eingesetzten Sorbit). Das erhaltene Produkt wurde vom Katalysator abfiltriert und eingeengt. Man erhielt 227 g eines Polyolgemischs aus 55% Sorbit, 40% Monoanhydrosorbit und 5% Dianhydrosorbit. Die Hydroxylzahl des Gemisches betrug 1602.

Beispiel 7

Beispiel 6 wurde wiederholt, wobei jedoch abweichend davon erst nach einer Reaktionszeit von 2 Stunden abfiltriert und eingeengt wurde. Man erhielt 222 g eines Polyolgemisches folgender Zusammensetzung: 40% Sorbit, 50% Monoanhydrosorbit und 10% Dianhydrosorbit; die Hydroxylzahl betrug 1500.

Beispiel 8

Beispiel 7 wurde mit einer auf 3 Stunden verlängerten Reaktionszeit wiederholt. Es wurden 216 g eines Polyolgemisches aus 20% Sorbit, 65% Monoanhydrosorbit und 15% Dianhydrosorbit mit einer Hydroxylzahl von 1374 erhalten.

Beispiel 9

Beispiel 8 wurde mit einer auf 4 Stunden verlängerten Reaktionszeit wiederholt. Nach Filtration und Destillation wurden 207 g eines Polyolgemisches aus 5% Sorbit, 60% Monoanhydrosorbit und 35% Dianhydrosorbit mit einer Hydroxylzahl von 1182 erhalten.

Beispiel 10

In einem 1 Ltr. Dreihalskolben wurden 357 g einer 70%igen wäßrigen Sorbitlösung mit 20 g des in Beispiel 1 beschriebenen Katalysators versetzt und 5 Stunden unter Rückflußbedingungen bei 125°C gerührt (Fremdwassergehalt 43%, bezogen auf eingesetzten Sorbit). Das erhaltene Reaktionsgemisch wurde vom katalysator abfiltriert und eingeengt. Man erhielt 232 g eines Polyolgemischs, das 70% Sorbit und 30% Monoanhydrosorbit enthielt. Die Hydroxylzahl betrug 1704.

Beispiel 11

Beispiel 10 wurde wiederholt, wobei jedoch abweichend davon 10 Stunden unter Rückflußbedingungen bei 125°C gerührt wurde. Nach Filtration und Einengen wurde ein Polyolgemisch aus 45% Sorbit, 50% Monoanhydrosorbit und 5% Dianhydrosorbit und der Hydroxylzahl 1554 erhalten; die Ausbeute betrug 225 g.

Beispiel 12

Beispiel 11 wurde mit einer Reaktionsdauer von 15 Stunden wiederholt. Das erhaltene Polyolgemisch (221 g) setzte sich aus 35% Sorbit, 57% Monoanhydrosorbit und 8% Dianhydrosorbit zusammen; es hatte eine Hydroxylzahl von 1488.

Beispiel 13

Beispiel 12 wurde mit einer Reaktionszeit von 20 Stunden wiederholt. Es wurden 217 g eines Polyolgemisches aus 20% Sorbit, 70% Monoanhydrosorbit und 10% Dianhydrosorbit mit der Hydroxylzahl 1404 erhalten.

Beispiel 14

In einem 1 Ltr. Dreihalskolben wurden 250 g Sorbit mit 167 g Wasser und 20 g des in Beispiel 1 angegebenen Katalysators 10 Stunden unter Rückflußbedingungen bei 125°C gerührt (Fremdwassergehalt 67%, bezogen auf eingesetzten Sorbit). Das Reaktionsgemisch wurde vom Katalysator abfiltriert und im Vakuum eingedampft. Es wurden 233 g eines Polyolgemisches aus 72% Sorbit und 28% Monoanhydrosorbit mit der Hydroxylzahl 1714 erhalten.

Beispiel 15

Beispiel 14 wurde mit einer Reaktionszeit von 20 Stunden wiederholt. Nach Filtrieren und Einengen wurden 225 g eines Polyolgemischs aus 44% Sorbit, 52% Monoanhydrosorbit und 4% Dianhydrosorbit erhalten, dessen Hydroxylzahl 1556 betrug.

Beispiel 16

Beispiel 15 wurde mit einer Reaktionszeit von 30 Stunden wiederholt. Es wurden 218 g eines Polyolgemischs aus 21% Sorbit, 72% Monoanhydrosorbit und 7% Dianhydrosorbit mit der Hydroxylzahl 1427 erhalten.

Beispiel 17

Beispiel 16 wurde mit einer Reaktionszeit von 40 Stunden wiederholt. Dabei wurden 210 g eines Polyolgemischs aus 86% Monoanhydrosorbit und 14% Dianhydrosorbit mit der Hydroxylzahl 1284 erhalten.

Beispeil 18

In einem 1 Ltr. Dreihalskolben wurden 250 g eines hydrierten Hemicellulosehydrolysates, das aus Maisschalen nach bekannten Verfahren gewonnen und unter heterogener Katalyse (Raney-Nickel, 25 atm $H_2$, 125°C, 50% Lösung, pH 5, 3 Stunden) hydriert wurde und 20 g eines stark sauren Ionenaustauschers in der $H^+$-Form (Polystyrolsulfonsäure-Gelharz, 2% DVB vernetzt) vorgelegt und 2 Stunden bei 130°C und unter Rückflußbedingungen gerührt.

Der Wassergehalt der Lösung war auf 10% eingestellt worden.

Das erhaltene Produkt wurde heiß vom Ionenaustauscher abfiltriert und durch Einengen wurden 235 g Produkt mit einer Hydroxylzahl von 1658 gegenüber 1790 beim Rohstoff erhalten.

Eine vergleichende HPLC-Studie zeigte, daß die im Rohstoff vorhandenen Polyole (zu 75% Pentite, davon über 55% Xylit, sowie Sorbit, Galaktit) zu 22% in Anhydroverbindungen umgesetzt waren.

Beispiel 19

Vorstehendes Beispiel wurde wiederholt, wobei abweichend die Reaktionszeit auf 4 Stunden verlängert wurde.

Nach entsprechender Aufarbeitung wurden 197 g Produkt mit einer Hydroxylzahl von 1115 erhalten.

Eine HPLC-Untersuchung ergabe eine Umsetzung der Rohstoffe in Anhydropolyole zu 85%.

Beispiel 20

Vorstehendes Beispiel wurde wiederholt, wobei abweichend die Reaktionszeit auf sechs Stunden verlängert und ein mit 10% DVB vernetztes Gelharz als Katalysator eingesetzt wurde.

Nach entsprechender Aufarbeitung wurden 193 g Produkt mit einer Hydroxylzahl von 1080 erhalten.

Eine HPLC-Untersuchung zeigte, daß die Polyole des Rohstoffs zu über 90% zu Anhydropolyolen umgesetzt waren.

Beispiel 21

Vorstehendes Beispiel 18 wurde wiederholt, wobei abweichend ein Wassergehalt von 19% in der Reaktionslösung eingestellt und als Katalysator ein mit 20% DVB vernetztes makroporöses Polystyrolsulfonsäureharz verwendet wurde.

Nach entsprechender Aufarbeitung wurden 236 g Produkt mit einer Hydroxylzahl von 1732 erhalten.

Eine HPLC-Untersuchung zeigte eine Umsetzung ca. 11% der Polyole zu Anhydropolyolen.

Beispiel 22

Vorstehendes Beispiel 21 wurde wiederholt, wobei abweichend eine Reaktionsdauer von vier Stunden angewandt wurde.

227 g Produkt mit einer Hydroxylzahl 1515 wurden erhalten, ein Umsatz von 38% der Polyole zu Anhydropolyolen ermittelt.

Beispiel 23

Vorstehendes Beispiel 22 wurde wiederholt, wobei abweichend eine Reaktionsdauer von sechs Stunden angewandt wurde.

210 g Produkt mit einer Hydroxylzahl 1327 wurden erhalten, ein Umsatz von 60% der Polyole zu Anhydropolyolen wurde ermittelt.

Beispiel 24

Vorstehendes Beispiel 22 wurde wiederholt, wobei abweichend eine Reaktionsdauer von acht Stunden angewandt wurde.

191 g Produkt mit einer Hydroxylzahl 1113 wurden erhalten, ein Umsatz von 87% der Polyole zu Anhydropolyolen wurde ermittelt.

**Patentansprüche**

1. Verfahren zur Herstellung von mindestens zwei Polyole verschiedenen Molekulargewichts aus den Gruppen der $C_4$—$C_6$-Zuckeralkohole und $C_4$—$C_6$-Anhydrozuckeralkohole der Formel

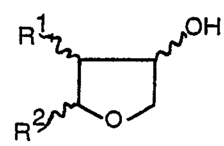

. (I)

in der $R^1$ eine Hydroxylgruppe oder das Äthersauerstoffatom eines zweiten Oxacyclopentanrings und $R^2$ ein Wasserstoffatom oder ein Hydroxymethyl- oder 1,2-Dihydroxyäthylrest ist oder zusammen mit $R^1$ und den Kohlenstoffatomen, an die $R^1$ und $R^2$ gebunden sind, einen zweiten Oxacyclopentanring bildet, enthaltenden Polyolgemisches, mit der Mäßgabe, daß mindestens ein Polyol der Formel I vorhanden ist, durch Waserabspaltung aus $C_4$—$C_6$-Zuckeralkohol(en) bei erhöhter Temperatur in flüssiger Phase und in Gegenwart eines stark-sauren heterogenen Katalysators, dadurch gekennzeichnet, daß man die Umsetzung

a) in Gegenwart von Wasser in einer Menge, die mindestens dem bei der Umsetzung frei werdenden Reaktionswasser und höchstens 120 Gew.-%, bezogen auf das Gewicht des eingesetzten Zuckeralkohol(gemisch)s, zugesetztem Wasser (Fremdwaser) entspricht,

b) bei einer Temperatur zwischen 60 und 200°C und

c) mit einer (durchschnittlichen) Reaktionszeit, die so gewählt ist, daß die Hydroxylzahl des erhaltenen Polyolgemisches in einem Bereich von 810 bis 1800 liegt und das Molverhältnis von Monoanhydrozuckeralkohol(en) zu Zuckeralkohol(en) und/oder Dianhydrohexit(en) mindestens 1:9 beträgt, durchführt.

2. Verfahren nach Anspruch 1, dadurch gekennzeichnet, daß die Umsetzung so lange fortgeführt wird, bis die Hydroxylzahl des erhaltenen Polyolgemisches in einem Bereich von 900 bis 1600, vorzugsweise 1100 bis 1500 und insbesondere 1150 bis 1400 liegt.

3. Verfahren nach Anspruch 1 oder 2, dadurch gekennzeichnet, daß die Umsetzung forgeführt wird, bis der Zuckeralkoholgehalt des erhaltenen Polyolgemisches höchstens 50, vorzugsweise höchstens 20 und insbesondere höchstens 10 Gew.-%, beträgt.

4. Verfahren nach einem der Ansprüche 1 bis 3, dadurch gekennzeichnet, daß der Wassergehalt des Reaktionsgemisches im Verlauf der Umsetzung variiert wird.

5. Verfahren nach einem der Ansprüche 1 bis 4, dadurch gekennzeichnet, daß die Umsetzung in Gegenwart von Wasser in einer Menge durchgeführt wird, die dem Reaktionswasser zuzüglich 0,1 bis 100, vorzugsweise 1 bis 70 und insbesondere 3 bis 50 Gew.-% Fremdwasser, bezogen auf eingesetzte(n) Zuckeralkohol(e), entspricht.

6. Verfahren nach einem der Ansprüche 1 bis 5, dadurch gekennzeichnet, daß die Umsetzung bei einer Temperatur im Bereich von 80 bis 170, vorzugsweise 95 bis 160 und insbesondere 105 bis 145°C, durchgeführt wird.

7. Verfahren nach einem der Ansprüche 1 bis 6, dadurch gekennzeichnet, daß als Katalysator ein stark saures Kationenaustauscherharz, stark saures anorganisches Molekularsieb und/oder ein saurer Crack- und/oder Hydrocrackkatalysator verwendet wird.

8. Verfahren nach Anspruch 7, dadurch gekennzeichnet, daß als Katalysator ein mit Divinylbenzol vernetztes Polystyrolsulfonsäure-Kationenaustauscherharz in der $H^+$-Form verwendet wird.

9. Verfahren nach einem der Ansprüche 1 bis 8, dadurch gekennzeichnet, daß die Umsetzung in einer Inertgasatmosphäre durchgeführt wird, wobei man vorzugsweise Inertgas durch das Reaktionsgemisch perlen läßt.

10. Verfahren nach einem der Ansprüche 1 bis 9, dadurch gekennzeichnet, daß die Umsetzung zumindest teilweise unter Rückflußbedingungen durchgeführt wird.

11. Verfahren nach einem der Ansprüche 1 bis 10, dadurch gekennzeichnet, daß ein organisches, mit Wasser zumindest teilweise mischbares und/oder azeotrop destillierbares (Hilfs-)Lösungsmittel verwendet wird.

12. Verfahren nach einem der Ansprüche 1 bis 11, dadurch gekennzeichnet, daß asl Ausgangsmaterial Sorbit, ein Sorbit enthaltendes Hexitgemisch oder ein mindestens zwei $C_4$—$C_6$-Zuckeralkohole mit unterschiedlichem Molekulargewicht enthaltendes Gemisch, insbesondere ein hydriertes Hemicelluloseprodukt, verwendet wird.

13. Verfahren nach einem der Ansprüche 1 bis 12, dadurch gekennzeichnet, daß man ein hexithaltiges Ausgangsmaterial verwendet und das zumindest in einer Phase, insbesondere der Anfangsphase der Umsetzung, auf einen einer Fremdwasserkonzentration von 1 bis 100, vorzugsweise 3 bis 70 und insbesondere 8 bis 50 Gew.-%, bezogen auf eingesetzte(n) Zuckeralkohol(e), entsprechenden Wassergehalt eingestellte Reaktionsgemisch so lange reagieren läßt, bis dessen Monoanhydrozuckeralkoholgehalt, bezogen auf Polyoltrockensubstanz, 35 bis 90, vorzugsweise 40 bis 85 und insbesondere 50 bis 80 Gew.-% beträgt und das Gewichtsverhältnis von Monoanhydro- zu Dianhydrohexit(en) in einem Bereich von 9:1 bis 1:2,8, vorzugsweise 4:1 bis 1:2 und insbesondere 2:1 bis 1:1 liegt.

14. Verfahren nach einem der Ansprüche 1 bis 13, dadurch gekennzeichnet, daß die Umsetzung kontinuierlich, vorzugsweise zwei- oder mehrstufig, durchgeführt wird.

**Revendications**

1. Procédé de production de mélanges de polyols contenant au moins deux polyols de poids moléculaires différents choisis dans les groupes de sucre-alcools en $C_4$ à $C_6$ et des anhydrosucre-alcools en $C_4$ à $C_6$, de formule

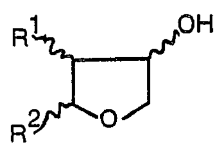

(I)

dans laquelle $R^1$ est un groupe hydroxyle ou représente l'atome d'oxygène d'éther d'un second noyau d'oxacyclopentane et $R^2$ est un atome d'hydrogène ou un reste hydroxyméthyle ou 1,2-dihydroxyéthyle ou forme conjointement avec $R^1$ et les atomes de carbone auxquels $R^1$ et $R^2$ sont liés, un second noyau d'oxacyclopentane, sous réserve qu'au moins un polyol de formule I soit présent, par élimination d'eau d'un ou plusieurs sucre-alcools en $C_4$ à $C_6$ à température élevée en phase liquide et en présence d'un catalyseur hétérogène fortement acide, caractérisé en ce qu'on conduit la réaction

a) en présence d'eau en une quantité qui correspond au moins à l'eau réactionnelle libérée lors de la réaction et d'eau ajoutée (eau étrangère), au maximum 120% en poids par rapport au poids du sucre-alcool (mélange) utilisé,

b) à une température comprise entre 60 et 200°C et

c) pendant une durée de réaction (moyenne) qui est choisie de manière que l'indice d'hydroxyle du mélange de polyols obtenu se situe dans un intervalle de 810 à 1800 et que le rapport molaire du ou des monoanhydrosucre-alcools au(x) sucre-alcool(s) et/ou au(x) dianhydrohexitol(s) s'élève au moins à 1:9.

2. Procédé suivant la revendication 1, caractérisé en ce qu'on poursuit la réaction jusqu'à ce que l'indice d'hydroxyle du mélange de polyols obtenu se situe dans un intervalle de 900 à 1600, de préférence de 1100 à 1500 et notamment de 1150 à 1400.

3. Procédé suivant la revendication 1 ou 2, caractérisé en ce qu'on poursuit la réaction jusqu'à ce que la teneur en sucre-alcools du mélange

de polyols obtenu s'élève au maximum à 50, de préférence qu maximum à 20 et notamment au maximum à 10% en poids.

4. Procédé suivant l'une des revendications 1 à 3, caractérisé en ce qu'on fait varier la teneur en eau du mélange réactionnel au cours de la réaction.

5. Procédé suivant l'une des revendications 1 à 4, caractérrisé en ce qu'on conduit la réaction en présence d'eau en une quantité qui correspond à l'eau de réaction, plus 0,1 à 100, de préférence 1 à 70 et notamment 3 à 50% en poids d'eau étrangère, par rapport au(x) sucre-alcool(s) utilisé(s).

6. Procédé suivant l'une des revendications 1 à 5, caractérisé en ce qu'on conduit la réaction à une température comprise dans l'intervalle de 80 à 170°C, de préférence de 95 à 160°C et notamment de 105 à 145°C.

7. Procédé suivant l'une des revendications 1 à 6, caractérisé en ce qu'on utilise comme catalyseur une résine d'échange cationique fortement acide, un tamis moléculaire inorganique fortement acide et/ou un catalyseur acide de craquage et/ou d'hydrocraquage.

8. Procédé suivant la revendication 7, caractérisé en ce qu'on utilise comme catalyseur une résine d'échange cationique du type acide polystyrènesulfonique réticulé au divinylbenzène, sous la forme $H^+$.

9. Procédé suivant l'une des revendications 1 à 8, caractérisé en ce qu'on conduit la réaction dans une atmosphère de gaz inerte, en faisant passer de préférence le gaz inerte en très fines bulles dans le mélange réactionnel.

10. Procédé suivant l'une des revendications 1 à 9, caractérisé en ce qu'on conduit la réaction au moins partiellement dans des conditions de reflux.

11. Procédé suivant l'une des revendications 1 à 10, caractérisé en ce qu'on utilise un solvant (auxiliaire) organique au moins partiellement miscible à l'eau et/ou susceptible de distillation azéotrope.

12. Procédé suivant l'une des revendications 1 à 11, caractérisé en ce qu'on utilise comme matière de départ du sorbitol, un mélange d'hexitols contenant du sorbitol ou un mélange contenant au moins deux sucre-alcools en $C_4$ à $C_6$ de poids moléculaires différents, notamment un produit formé d'hémicellulose hydrogènée.

13. Procédé suivant l'une des revendications 1 à 12, caractérisé en ce qu'on utilise une matière de départ contenant des hexitols et on fait réagir le mélange réactionnel réglé tout au moins dans une phase, notamment dans la phase initiale de la réaction, à une teneur en eau correspondant à une concentration en eau étrangère de 1 à 100, de préférence 3 à 70 et notamment 8 à 50% en poids par rapport au(x) sucre-alcool(s) utilisé(s) jusqu'à ce que sa teneur en mono-anhydrosucre-alcools, par rapport aux polyols sur base sèche, s'élève à 35—90, de préférence à 40—85 et notamment à 50—80% en poids et que le rapport en poids mono-anhydrohexytol(s): dianhydrohexytol(s) se situe dans un intervalle de 9:1 à 1:2,8, de préférence de 4:1 à 1:2 et notamment de 2:1 à 1:1.

14. Procédé suivant l'une des revendications 1 à 13, caractérisé en ce qu'on conduit la réaction en continu, de préférence en deux ou plus de deux étapes.

**Claims**

1. A process for producing a polyol mixture containing at least two polyols of different molecular weights from the groups of $C_4$—$C_6$ sugar alcohols and $C_4$—$C_6$-anhydrosugar alcohols of the formula

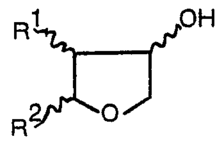

(I)

in which $R^1$ is a hydroxyl group or the ether oxygen atom of a second oxacyclopentane ring and $R^2$ is a hydrogen atom or a hydroxymethyl- or 1,2-dihydroxyethyl group or forms, together with $R^1$ and the carbon atoms to which $R^1$ and $R^2$ are bonded, a second oxacyclopentane ring, provided that at least one polyol of formula 1 is present, by dehydration of $C_4$—$C_6$ sugar alcohol(s) at a high temperature in the liquid phase and in the presence of a heterogeneous strong acid catalyst, characterized in that conversion is effected

a) in the presence of water in a quantity which corresponds at least to the water of reaction released on conversion and at most to 120% by weight, based on the weight of the sugar alcohol (mixture) used, of added water (foreign water),

b) at a temperature of between 60 and 200°C, and

c) with an (average) reaction time which is so selected that the hydroxyl value of the polyol mixture obtained lies in a range of from 810 to 1800 and the mole ratio of monoanhydrosugar alcohol(s) to sugar alcohol(s) and/or dianhydrohexitol(s) amounts to at leats 1:9.

2. A process according to claim 1, characterized in that conversion is carried out until the hydroxyl value of the polyol mixture obtained ranges from 900 to 1600, preferably 1100 to 1500, and particularly 1150 to 1400.,

3. A process according to claim 1 or claim 2, characterized in that conversion is effected until the sugar alcohol content of the polyol mixture obtained amounts to 50 at the most, preferably to 20 at the most and especially to no more than 10% by weight.

4. A process according to any one of claims 1 to 3, characterized in that the water content of the reaction mixture is varied in the course of conversion.

5. A process according to any one of claims 1 to 4, characterized in that conversion is carried out in the presence of water in a quantity which corresponds to the water of reaction plus 0.1 to 100, preferably 1 to 70 and especially 3 to 50% by weight added water, based on sugar alcohol(s) used.

6. A process according to any one of claims 1 to 5, characterized in that conversion is effected at a temperature ranging from 80 to 170, preferably 95 to 160 and especially 105 to 145°C.

7. A process according to any one of claims 1 to 6, characterized in that the catalyst used is a strongly acid cation exchange resin, a strongly acid inorganic molecular sieve and/or an acid crack- and/or hydrocrack catalyst.

8. A process according to claim 7, characterized in that the catalyst used is a polystyrene sulphonic acid cation exchange resin in $H^+$ form cross-linked with divinyl benzol.

9. A process according to any one of claims 1 to 8, characterized in that conversion is carried out in an inert gas atmosphere, inert gas preferably being allowed to bubble through the reaction mixture.

10. A process according to any one of claims 1 to 9, charcterized in that conversion is carried out at least partially under reflux conditions.

11. A process according to any one of claims 1 to 10, characterized in that an organic (auxiliary) solvent which may be at least partially mixed and/or azeotropically distilled with water is used.

12. A process according to any one of claims 1 to 11, characterized in that the starting material used is sorbitol, a hexitol mixture containing sorbitol or a mixture containing at least two $C_4$—$C_6$ sugar alcohols with different molecular weights, especially a hydrogenated hemicellulose product.

13. A process according to any one of claims 1 to 12, characterized in that a hexitol-containing starting material is used and in that the reaction mixture, which is adjusted in at least one phase, especially the starting phase of conversion, to a water content corresponding to an added water concentration of from 1 to 100, preferably 3 to 70 and especially 8 to 50% by weight, based on the sugar alcohol(s) used, is allowed to react until its monoanhydrosugar alochol content, based on polyol solids, amounts to from 35 to 90, preferably 40 to 85 and especially 50 to 80% by weight and the weight ratio of monoanhydro- to dianhydrohexitol(s) ranges from 9:1 to 1:2.8, preferably 4:1 to 1:2 and especially 2:1 to 1:1.

14. A process according to any one of claims 1 to 13, characterized in that conversion is effected continuously, preferably in two or more stages.

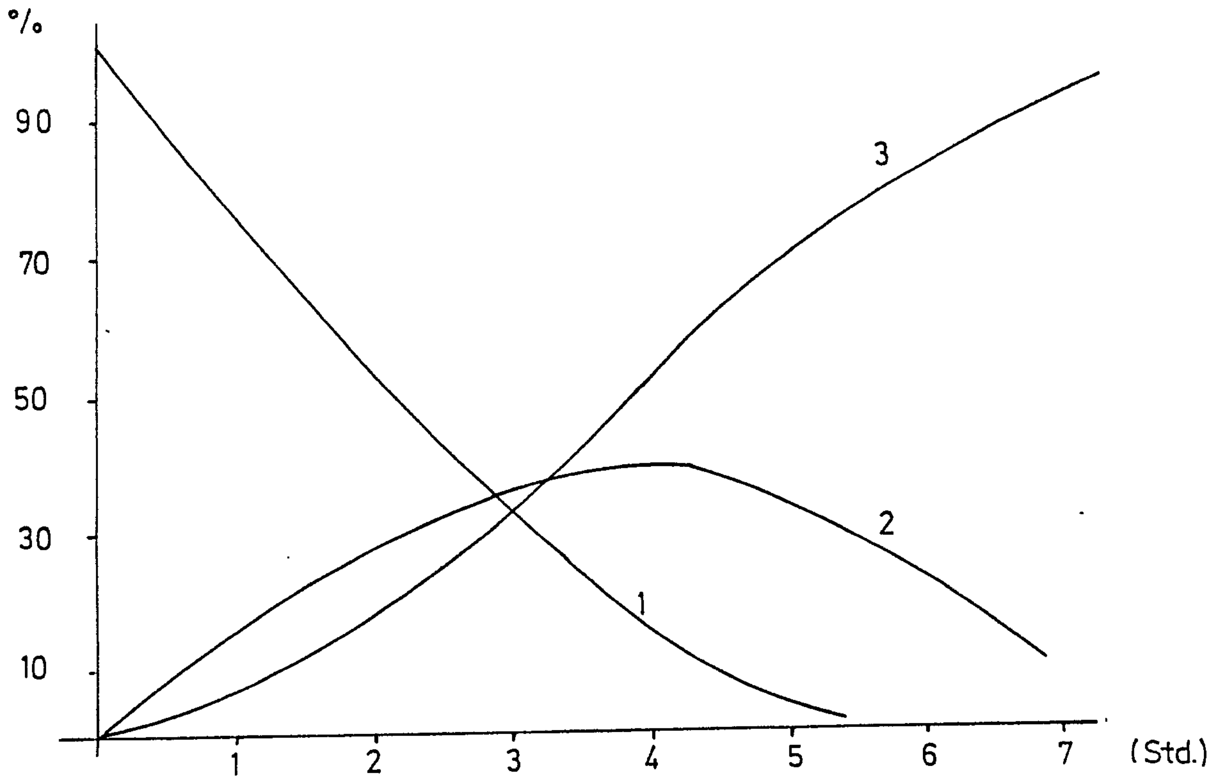


1 Sorbit
2 Monoanhydrosorbit
3 Dianhydrosorbit

Fig. 1

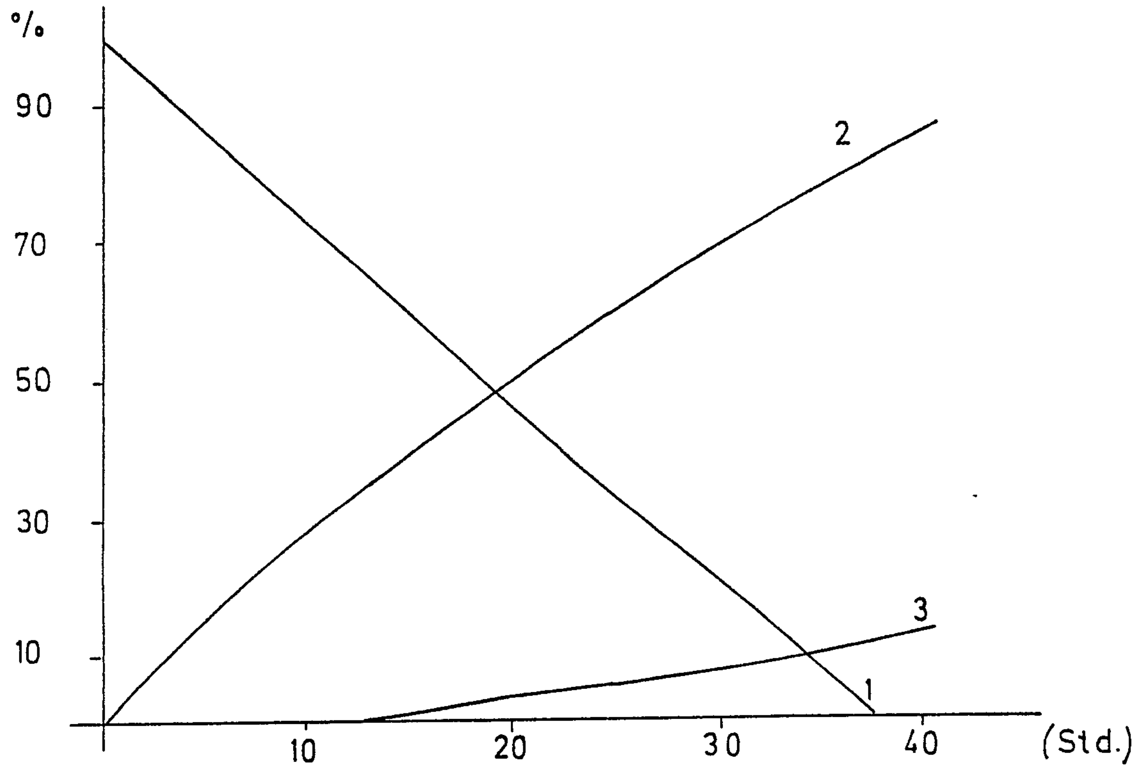


Fig. 2

1 Sorbit
2 Monoanhydrosorbit
3 Dianhydrosorbit